(19) Europäisches Patentamt
European Patent Office
Office européen des brevets

(11) **EP 1 626 945 B1**

(12) **EUROPÄISCHE PATENTSCHRIFT**

(45) Veröffentlichungstag und Bekanntmachung des Hinweises auf die Patenterteilung:
**13.08.2008 Patentblatt 2008/33**

(21) Anmeldenummer: 04739206.3

(22) Anmeldetag: **14.05.2004**

(51) Int Cl.:
*C07C 7/13* (2006.01)          *C07C 11/09* (2006.01)

(86) Internationale Anmeldenummer:
**PCT/EP2004/005200**

(87) Internationale Veröffentlichungsnummer:
**WO 2004/101477 (25.11.2004 Gazette 2004/48)**

(54) **VERRINGERUNG DES GEHALTS AN SAUERSTOFFHALTIGEN UND/ODER STICKSTOFFHALTIGEN VERBINDUNGEN IN ISOBUTENHALTIGEN STOFFSTRÖMEN**

REDUCTION OF THE CONTENT OF COMPOUNDS CONTAINING OXYGEN AND/OR NITROGEN IN MATERIAL FLOWS CONTAINING ISOBUTENE

DIMINUTION DE LA TENEUR EN COMPOSES CONTENANT DE L'OXYGENE ET/OU DE L'AZOTE DANS DES FLUX DE MATIERES CONTENANT DE L'ISOBUTENE

(84) Benannte Vertragsstaaten:
**AT BE BG CH CY CZ DE DK EE ES FI FR GB GR HU IE IT LI LU MC NL PL PT RO SE SI SK TR**

(30) Priorität: **16.05.2003 DE 10322153**

(43) Veröffentlichungstag der Anmeldung:
**22.02.2006 Patentblatt 2006/08**

(73) Patentinhaber: **BASF SE**
**67056 Ludwigshafen (DE)**

(72) Erfinder:
• **WETTLING, Thomas**
**67117 Limburgerhof (DE)**
• **BORCHERS, Dirk**
**67067 Ludwigshafen (DE)**

(74) Vertreter: **Reitstötter - Kinzebach**
**Patentanwälte**
**Sternwartstrasse 4**
**81679 München (DE)**

(56) Entgegenhaltungen:
**EP-A- 0 582 901          DE-A- 3 914 817**
**US-A- 5 120 881          US-A- 5 427 689**

• **DATABASE WPI Section Ch, Week 8409 Derwent Publications Ltd., London, GB; Class A41, AN 1984-054362 XP002302096 & SU 1 011 624 A (GEOR. OIL RES. INST.) 15. April 1983 (1983-04-15) in der Anmeldung erwähnt**

**Beschreibung**

Die vorliegende Erfindung betrifft ein Verfahren zur Verringerung des Gehalts an sauerstoffhaltigen und/oder stickstoffhaltigen Verbindungen in Stoffströmen mit einem Isobuten-Gehalt von wenigstens 10 Gew.-%.

Bei der thermischen oder thermisch/katalytischen Spaltung von Erdgas, Raffineriegas oder bestimmten Erdölfraktionen, wie sie z. B. in Steamcrackern erfolgt, fallen nach der Aufarbeitung der Spaltprodukte unter anderem Gemische aus Butadien, Butanen, n-Butenen und Isobuten an, die allgemein als $C_4$-Schnitte bezeichnet werden.

Diese $C_4$-Schnitte enthalten neben den $C_4$-Kohlenwasserstoffen Spuren verschiedener sauerstoffhaltiger und/oder stickstoffhaltiger Verbindungen. Die Art und Menge dieser Verunreinigungen hängen von der Art und dem Ursprung des eingesetzten Rohstoffs und von den technischen Bedingungen der Spaltungsreaktion ab. Auch die weitere Aufarbeitung des $C_4$-Schnitts beeinflusst den Gehalt an diesen Verunreinigungen.

Eine Verwendungsmöglichkeit von $C_4$-Schnitten, die zunehmend Bedeutung findet, ist die Polymerisation des im $C_4$-Schnitt enthaltenen Isobutens zu Isobutenhomo- oder -copolymeren. Solche Polymerisationsverfahren sind beispielsweise in der WO 93/10063 oder der EP 0 628 575 B1 beschrieben.

Die Anwesenheit von sauerstoffhaltigen und/oder stickstoffhaltigen Verbindungen im Ausgangsmaterial der Polymerisation ist unerwünscht, denn sie führt zur Desaktivierung der verwendeten Katalysatoren bzw. einem übermäßigen Katalysatorverbrauch.

Es ist bekannt, Katalysatorgifte durch Adsorption an feste Adsorbentien wie Molekularsiebe bzw. Zeolithe zu entfernen. So lehrt die DE-A 39 14 817, ein Einsatz-Kohlenwasserstoffgemisch vor seiner Oligomerisierung über ein Molekularsieb mit einem Porendurchmesser von mehr als 4 bis 15 Angström zu leiten.

Die SU-A 1011624 beschreibt ein Verfahren zur Abtrennung von Carbonylverunreinigungen aus gasförmigen Isobuten-Strömen durch Adsorption an einen Magnesium-A-Zeolithen. Die EP-A 582 901 beschreibt die Abtrennung von Acetaldehyd von $C_3$-$C_{15}$-Kohlenwasserstoffen, wie Isobuten, indem man den Kohlenwasserstoff in flüssiger Phase mit einem Zeolithen in Kontakt bringt, dessen Porenweite größer als 0,3 nm und bis zu 0,75 nm ist.

Stoffströme, die Isobuten enthalten, können nicht ohne Weiteres durch Behandlung mit festen Adsorbentien gereinigt werden. Isobuten bildet - an aktiven Stellen der Oberfläche des Adsorbens - sehr leicht ein tertiäres Carbokation, an das ein weiteres Isobutenmolekül addiert werden kann. Letztlich entsteht unter Rückbildung der Doppelbindung ein Isooctenmolekül bzw. nach Addition weiterer Isobutenmoleküle Tri- oder höhere Oligomere des Isobutens. Die Bildung von Isoocten oder höheren Oligomeren des Isobutens ist wegen des Verlusts an Isobuten unerwünscht. Außerdem beeinträchtigt ein hoher Gehalt an Isooctenen oder höheren Oligomeren des Isobutens das Polymerisationsverhalten, da diese zum vorzeitigen Kettenabbruch führen und/oder den Gehalt an Polyisobutenmolekülen mit endständigen Vinylidendoppelbindungen verringern. Ersteres erschwert die Herstellung mittel- oder hochmolekularer Isobutenpolymere, letzteres die Herstellung so genannter hochreaktiver Polyisobutene, d. h. solcher mit hohem Gehalt an Vinylidendoppelbindungen.

Der Erfindung liegt daher die Aufgabe zugrunde, ein Verfahren zur Verringerung des Gehalts an sauerstoffhaltigen und/oder stickstoffhaltigen Verbindungen in Stoffströmen mit einem Isobuten-Gehalt von wenigstens 10 Gew.-% bereitzustellen, bei dem die Bildung von Isooctenen und höheren Oligomeren des Isobutens unterdrückt ist.

Erfindungsgemäß wird diese Aufgabe gelöst, indem man den Stoffstrom in flüssiger Phase bei einer Temperatur T [in K] und mit einer linearen Geschwindigkeit v [in cm/min] über ein Festbett eines säurefreien Zeoliths einer mittleren Porengröße von 0,3 bis 1,5 nm, vorzugsweise 0,5 bis 1,2 nm, leitet, wobei das Festbett in Strömungsrichtung des Stoffstroms eine Ausdehnung l [in cm] aufweist und T, v und l der Gleichung

$$2^{(T-283\ K)/10\ K} \bullet l/v \leq 500\ \text{min}$$

vorzugsweise

$$2^{(T-283\ K)/10\ K} \bullet l/v \leq 350\ \text{min}$$

gehorchen.

Mit der Erfindung wurde gefunden, dass die Bildung von Isobutenoligomeren wie Isooctenen durch Wahl eines Zeolithen geeigneter Porengröße und Kontrolle der Temperatur und der Kontaktzeit, die ihrerseits von der linearen Geschwindigkeit des Stoffstroms und der Bettausdehnung des Zeolith-Festbetts abhängt, unterdrückt werden kann.

Als lineare Geschwindigkeit bzw. Leerrohrgeschwindigkeit v des Stoffstroms wird das Verhältnis des Volumenstroms [in cm$^3$/min] zum Querschnitt des Zeolith-Festbetts [in cm$^2$] verstanden. Vorzugsweise liegt v im Bereich von 0,5 bis 35 cm/min, insbesondere 1 bis 15 cm/min, besonders bevorzugt 1,5 bis 10 cm/min.

Vorzugsweise beträgt T weniger als 40 °C, insbesondere liegt T im Bereich - 30 bis 30 °C, besonders bevorzugt - 25 bis 20 °C.

Der Druck wird so gewählt, dass der Stoffstrom in flüssiger Phase vorliegt. Der Druck beträgt im Allgemeinen 1 bis 70 bar, vorzugsweise 5 bis 35 bar. Es ist zweckmäßig, bei dem Druck zu arbeiten, bei dem der Stoffstrom bei der Herstellung anfällt oder gelagert, transportiert oder weiterverwendet wird.

Der erfindungsgemäß behandelte Stoffstrom enthält wenigstens 10 Gew.-% Isobuten, vorzugsweise wenigstens 20 Gew.-% und insbesondere wenigstens 40 Gew.-%. Es kann sich bei dem Stoffstrom auch um im Wesentlichen reines Isobuten handeln, d. h. Ströme mit mehr als 99 Gew.-% Isobuten.

Typische sauerstoffhaltige und stickstoffhaltige Verbindungen in den Stoffströmen, die erfindungsgemäß behandelt werden, sind Aldehyde wie Acetaldehyd, Ketone wie Aceton, Alkohole wie Methanol, Ethanol, tert-Butanol, Ether wie Methyl-tert-butylether, I-sopropyl-tert-Butylether, Isobutyl-tert-butylether, Nitrile wie Acetonitril. Obgleich nach dem erfindungsgemäßen Verfahren Stoffströme mit Konzentrationen an sauerstoffhaltigen und stickstoffhaltigen Verbindungen von bis zu 5 Gew.-% behandelt werden können, ist die Behandlung von Stoffströmen mit mehr als 1000 ppm dieser Verbindungen aufgrund des damit verbundenen hohen Verbrauchs an Zeolith bzw. der häufig erforderlichen Regeneration des Zeolithen nicht zweckmäßig. In diesen Fällen ist es günstiger, die Konzentration der sauerstoffhaltigen und stickstoffhaltigen Verbindungen zuvor mit konventionellen Verfahren auf weniger als 1000 ppm zu verringern. Typischerweise enthalten die Stoffströme, die nach dem erfindungsgemäßen Verfahren behandelt werden, 10 bis 500 ppm sauerstoffhaltige und stickstoffhaltige Verbindungen.

In bevorzugten Ausführungsformen enthält der Stoffstrom außerdem von Isobuten verschiedene Kohlenwasserstoffe, z. B. $C_2$-$C_8$-Kohlenwasserstoffe, insbesondere $C_4$ Kohlenwasserstoffe. So kann der Stoffstrom gesättigte Kohlenwasserstoffe, wie Pentan, Hexan, Cyclohexan, Methylcyclohexan oder Isooctan, oder ungesättigte Kohlenwasserstoffe, wie Ethen oder lineare Butene, enthalten. Als Einsatzstoffe für das erfindungsgemäße Verfahren kommen z. B. Butadien-arme $C_4$-Raffinate aus Crackern oder $C_4$-Schnitte der Isobutan-Dehydrierung in Frage.

In anderen Ausführungsformen enthält der Stoffstrom außerdem halogenierte Kohlenwasserstoffe, insbesondere chlorierte Kohlenwasserstoffe wie Chlormethan, Dichlormethan, Dichlorethan oder Trichlormethan, die als Reaktionslösungsmittel für eine anschließende Polymerisation dienen.

Zeolithe, die auch als Molekularsiebe bezeichnet werden, sind kristalline Aluminosilikate, die ein hochgeordnetes Gerüst mit einem starren dreidimensionalen Netzwerk von $SiO_4$ und $AlO_4$-Tetraedern aufweisen, welche durch gemeinsame Sauerstoffatome verbunden sind. Zur Kompensation der Elektrovalenz der Aluminium enthaltenden Tetraeder enthalten die Zeolithe Kationen. Das Aluminium im Gitter der Zeolithe kann ganz oder teilweise durch andere Elemente wie B, Ga, Fe, Cr, V, As, Sb, Bi oder Be oder Gemische davon ersetzt sein. Das Silicium kann durch ein anderes vierwertiges Element, z. B. Ge, Ti, Zr oder Hf, ersetzt sein.

Der erfindungsgemäß verwendete Zeolith ist säurefrei, d. h. er enthält zur Ladungskompensation der negativen Gerüstladung keine Protonen. Vorzugsweise enthält der Zeolith Natriumionen und/oder Erdalkaliionen wie Calciumionen zur Ladungskompensation. Beim Kontakt mit bestimmten Chemikalien, wie Chloriden oder Fluoriden, kann die Oberfläche des Zeoliths unter Ausbildung saurer Zentren dauerhaft verändert werden. Der Kontakt mit solchen Chemikalien sollte daher vermieden werden. Am besten setzt man für das erfindungsgemäße Verfahren fabrikfrischen Zeolith ein. Wenn die irreversible Kontamination ein bestimmtes Maß übersteigt, sollte der Zeolith ersetzt werden.

Zeolithe können nach bekannten Verfahren synthetisch hergestellt werden, vgl. z. B. Ullmanns Enzyklopädie d. Techn. Chemie, 4. Aufl. Bd. 17 S. 9-17 (1983). Die Zeolithe können in eine oxidische Bindemittelmatrix, z. B. aus Aluminiumoxiden, Siliciumdioxid, Gemischen von hochdispersem Siliciumdioxid und Aluminiumoxid, Titandioxid, Zirkondioxid oder Ton eingebettet und zu Förmkörpern wie Strängen oder Tabletten geformt werden.

Im erfindungsgemäßen Verfahren werden Zeolithe mit mittleren Porengrößen von 0,3 bis 1,5 nm eingesetzt. Die mittlere Porengröße ist durch den Kristallaufbau festgelegt und kann z. B. aus Röntgenstrukturdaten ermittelt werden. In Zeolithe mit kleineren mittleren Porengrößen können sauerstoffhaltige und/oder stickstoffhaltige Verbindungen schlecht eindiffundieren und werden daher unzureichend adsorbiert. Zeolithe mit größeren mittleren Porengrößen führen beim Kontakt mit den isobutenhaltigen Stoffströmen zur vermehrten Bildung von Isobutenoligomerisierungsprodukten.

Bevorzugte Zeolithe sind unter Zeolith A, Zeolith L, Zeolith X und Zeolith Y ausgewählt. Natrium-Zeolith A oder Natrium-Zeolith A, in dem die Natriumionen ganz oder teilweise durch Calciumionen ersetzt sind, ist besonders bevorzugt.

Es ist bisweilen vorteilhaft, den Stoffstrom vor der Zeolithbehandlung zu trocknen und den Wassergehalt des Stoffstroms z. B. auf weniger als 5 ppm, vorzugsweise weniger als 1 ppm zu verringern. In einer bevorzugten Ausführungsform verwendet man eine strukturierte Schüttung zweier unterschiedlicher Zeolithe. So leitet man den Stoffstrom über ein Festbett, das stromaufwärts zur Strömungsrichtung des Stoffstromes einen Zeolith mit einer mittleren Porengröße von etwa 0,3 bis 0, 4 nm und stromabwärts einen Zeolith mit einer mittleren Porengröße von wenigstens 0,5 nm umfasst. Der Stoffstrom kommt in einer ersten Zone zuerst mit dem Zeolith geringer Porengröße in Kontakt, wobei bevorzugt Wasser adsorbiert wird. Größere sauerstoff- oder stickstoffhaltige Moleküle werden in dieser ersten Zone mit geringerer Präferenz adsorbiert. Erst in der anschließenden zweiten Zone werden diese von dem großporigeren Zeolith adsorbiert. Wasser hat eine höhere Affinität zu Zeolithen als größere sauerstoff- oder stickstoffhaltige Moleküle. Die beschriebene Ausführungsform, in der in einer ersten Zone bevorzugt Wasser entfernt wird, weist den Vorteil auf, dass - auch bei fortgeschrittener Sättigung des Zeolithen - keine Verdrängung bereits adsorbierter sauerstoff- oder stickstoffhaltiger Moleküle durch Wasser erfolgt.

Der Zeolith oder die Kombination von Zeolithen liegt in einer Festbettschüttung vor, die in einer Adsorptionssäule angeordnet ist, durch die der Stoffstrom geleitet wird. Die Adsorptionssäule ist vorzugsweise vertikal angeordnet und wird vom Stoffstrom in Richtung der Schwerkraft oder entgegen der Schwerkraft durchströmt. Die Ausdehnung des Festbetts in Strömungsrichtung beträgt vorzugsweise das 2- bis 15-fache des (längsten) Durchmessers des Festbetts. Es können auch mehrere hintereinandergeschaltete Adsorptionssäulen verwendet werden, die z. B. mit unterschiedlichen Zeolithen gefüllt sein können.

Nach einer Betriebsdauer ist der Zeolith gesättigt, d. h. die seine Oberfläche ist mit sauerstoffhaltigen und/oder stickstoffhaltigen Verbindungen belegt und beim Durchleiten des Stoffstroms findet keine weitere adsorptive Entfernung dieser Verbindungen aus dem Stoffstrom statt. Der Zeolith kann durch Überleiten eines Inertgases wie Stickstoff bei erhöhter Temperatur, z. B. von 150 bis 250 °C und bei Umgebungsdruck oder unter Vakuum, regeneriert werden. Ein typischer Regenerationszyklus dauert etwa 4 bis 24 Stunden. Zweckmäßigerweise sieht man wenigstens zwei Adsorptionssäulen vor, von denen sich eine erste Säule im Adsorptionszyklus befindet, während die andere Säule regeneriert wird. Ist der Zeolith der ersten Säule gesättigt, wird der Stoffstrom umgeleitet und über die zweite Adsorptionssäule geleitet, so dass der Zeolith in der ersten Säule regeneriert werden kann.

Nach der erfindungsgemäßen Zeolith-Behandlung weisen die Stoffströme in der Regel eine Gesamtkonzentration an sauerstoff- und stickstoffhaltigen Verbindungen von weniger als 20 ppm, insbesondere weniger als 10 ppm auf, wobei die Konzentrationen der einzelnen Kontaminanten im Allgemeinen jeweils weniger als 2 ppm, vorzugsweise weniger als 1 ppm betragen. Die erfindungsgemäß behandelten Stoffströme eignen sich besonders zur Herstellung von Isobutenpolymeren. Die Herstellung von Isobutenhomo- und -copolymeren ist an sich bekannt, vgl. z. B. WO 93/10063 oder EP 0 628 575 B1. Als Comonomere kommen vor allem konjugierte Diene wie Butadien und Isopren oder vinylaromatische Verbindungen wie Styrol in Betracht.

Als Polymerisationskatalysatoren zur Herstellung von Isobutenhomopolymeren werden vorzugsweise solche auf Bortrifluorid-Basis verwendet, insbesondere Bortrifluorid-Komplexe mit wenigstens einem unter Wasser, primären $C_1$-$C_5$-Alkanolen, sekundären $C_3$-$C_5$-Alkanolen und/oder Ethern ausgewählten Cokatalysator verwendet. Als Cokatalysatoren sind beispielsweise Wasser, Methanol, Ethanol, 2-Propanol, 1-Propanol und/oder tert-Butylmethylether geeignet. Die Bortrifluorid-Komplex-Katalysatoren können vor ihrem Einsatz vorgeformt werden oder in situ im Polymerisationsreaktor erzeugt werden. Pro mol Olefinmonomere setzt man vorzugsweise 0,1 bis 25 mmol, insbesondere 0,5 bis 10 mmol Komplexkatalysator, berechnet als Bortrifluorid, ein.

Die Polymerisation des Isobutens erfolgt vorzugsweise nach einem kontinuierlichen Verfahren. Dazu kann in herkömmlichen Reaktoren, wie Rohrreaktoren, Rohrbündelreaktoren oder Rührkesseln gearbeitet werden. Vorzugsweise erfolgt die Polymerisation in einem Schlaufenreaktor, also einem Rohr- oder Rohrbündelreaktor mit stetigem Umlauf des Reaktionsgutes, wobei in der Regel das Verhältnis von Zulauf zu Umlauf Z/U im Bereich von 1:5 bis 1:500, vorzugsweise im Bereich von 1:10 bis 1:200 v/v, variiert werden kann.

Die Polymerisation erfolgt zweckmäßigerweise bei Temperaturen unterhalb 0 °C, vorzugsweise bei Temperaturen im Bereich von 0 bis -40 °C, insbesondere im Bereich von 0 bis -30 °C und besonders bevorzugt im Bereich von -10 bis -30 °C. In der Regel wird die Polymerisation bei einem Druck im Bereich von 0,5 bis 20 bar (absolut) durchgeführt.

Die Verweilzeit des zu polymerisierenden Isobutens im Reaktor liegt je nach Reaktionsbedingungen und gewünschten Eigenschaften des herzustellenden Polymerisats im Bereich von 1 bis 120 Minuten, vorzugsweise im Bereich von 5 bis 60 Minuten.

Zur Aufarbeitung wird der Reaktionsaustrag zweckmäßigerweise in ein Medium geleitet, das den Polymerisationskatalysator desaktiviert und auf diese Weise die Polymerisation abbricht. Dazu können beispielsweise Wasser, Alkohole, Ether, Acetonitril, Ammoniak, Amine oder wässrige Lösungen von Mineralbasen, wie Alkalimetall- und Erdalkalimetall-Hydroxidlösungen, Lösungen von Carbonaten dieser Metalle und dergleichen verwendet werden. Bevorzugt wird ein Abbruch mit Wasser bei einer Temperatur von 20 bis 40 °C, beispielsweise in Form einer Druckwäsche. Im weiteren Gang der Aufarbeitung wird das Polymerisationsgemisch gegebenenfalls einer oder mehreren Extraktionen zur Entfernung von Restmengen an Katalysator - üblicherweise Methanol- oder Wasserwäschen - unterworfen. Anschließend werden destillativ nicht umgesetztes Isobuten, Lösungsmittel und flüchtige Isobutenoligomere abgetrennt. Der Sumpf wird von Resten des Lösungsmittels und Monomeren, beispielsweise über Dünnschichtverdampfer,- Fallfilmverdampfer, Ringspaltverdampfer oder Sambay-Verdampfer, befreit, gegebenenfalls unter Zusatz von Wasserdampf oder Stickstoffgas.

Die Erfindung wird durch die folgenden Beispiele näher veranschaulicht.

Beispiele 1 bis 6 und Vergleichsbeispiele 7 und 8

Isobutenhaltige Kohlenwasserstoffe mit bestimmten Konzentrationen an sauerstoffhaltigen und stickstoffhaltigen Verunreinigungen wurden über mit Zeolith gefüllte Säulen geleitet. Anschließend wurden die Konzentration der sauerstoffhaltigen bzw. stickstoffhaltigen Verunreinigungen und der Gehalt an Isooctenen gemessen. Die Ergebnisse sind in der nachstehenden Tabelle zusammengefasst, wobei alle Prozentangaben Gew.-% und alle ppm-Angaben Gew.-ppm (bestimmt durch GC-Analytik) sind. In der Tabelle werden folgende Abkürzungen verwendet: MTBE = Methyl-tert-butylether; IPTBE = Isopropyl-tert-butylether; IBTBE = Isobutyl-tert-butylether.

UOP 3A (EPG 1/16) ist ein Kalium-Zeolith A mit einer mittleren Porengröße von 0,3 nm der Firma UOP. UOP 5A ist ein Calcium-Zeolith A mit einer mittleren Porengröße von 0,5 nm. Grace 10A ist ein Zeolith A mit einer mittleren Porengröße von 1,0 nm der Firma Grace.

Es wurden zwei unterschiedliche Adsorberkolonnen verwendet (Länge/Durchmesser): Kolonne A: 6000 mm/1500 mm; Kolonne B: 6400 mm/2100 mm

| Bsp. | Kolonne | Zeolith | Menge | Kohlen-wasserstoff | Verunreinigungen/Gehalt | | | Temperatur | Druck | Leerrohr-geschw. | Isobutendimere (ppm) | | |
|---|---|---|---|---|---|---|---|---|---|---|---|---|---|
| | | | | | Art | (vor) | (nach) | | | | vor | nach | Differenz |
| 1 | A | UOP 5A | 6 t | Isobuten 17,30%<br>1-Buten 0,80%<br>2-Butene 1,10%<br>Hexan 80,40% | Methanol<br>Isopropanol<br>MTBE<br>IPTBE<br>IBTBE | 57 ppm<br>17 ppm<br>13 ppm<br>17 ppm<br>8 ppm | 3 ppm<br><1 ppm<br><1 ppm<br><1 ppm<br><1 ppm | 7 °C | 11 bar | 6,9 cm/min | 2315 | 2353 | 38 |
| 2 | A | UOP 5A | 6 t | Isobuten 17,10%<br>1-Buten 0,79%<br>2-Butene 1,17%<br>Hexan 80,47% | Methanol<br>Isopropanol<br>MTBE<br>IPTBE<br>IBTBE | 51 ppm<br>16 ppm<br>13 ppm<br>17 ppm<br>5 ppm | 4 ppm<br><1 ppm<br><1 ppm<br><1 ppm<br><1 ppm | 7 °C | 11 bar | 9,5 cm/min | 2127 | 2141 | 14 |
| 3 | A | UOP 3A<br>UOP 5A | 2 t<br>4 t | Isobuten 17,00%<br>1-Buten 0,83%<br>2-Butene 0,96%<br>Hexan 80,24% | Methanol<br>Isopropanol<br>MTBE<br>IPTBE<br>IBTBE | 47 ppm<br>9 ppm<br>28 ppm<br>13 ppm<br>11 ppm | <1 ppm<br><1 ppm<br><1 ppm<br><1 ppm<br><1 ppm | 7 °C | 11 bar | 7,9 cm/min | 1248 | 1257 | 9 |
| 4 | B | UOP 3A<br>Grace 10A | 4 t<br>8 t | Isobuten >99%<br>1-Buten 0,10%<br>2-Butene 0,20% | Acetonitril<br>Acetaldehyd<br>Methanol<br>Ethanol<br>Aceton | 7 ppm<br>2 ppm<br>9 ppm<br>17 ppm<br>58 ppm | <1 ppm<br><1 ppm<br><1 ppm<br><1 ppm<br><1 ppm | 9 °C | 14 bar | 2,6 cm/min | <10 | 37 | max. 37 |

| 5 | B | UOP 3A Grace 10A | 4 t 8 t | Isobuten >99% 1-Buten 0,10% 2-Butene 0,20% | Acetonitril 7 ppm <1 ppm Acetaldehyd 2 ppm <1 ppm Methanol 11 ppm <1 ppm Ethanol 18 ppm <1 ppm Aceton 58 ppm <1 ppm | 9 °C | 14 bar | 2,1 cm/min | <10 | 43 | max. 43 |
| 6 | B | Grace 10A | 12 t | Isobuten >99% 1-Buten 0,10% 2-Butene 0,20% | Acetonitril 7 ppm <1 ppm Acetaldehyd 2 ppm <1 ppm Methanol 11 ppm <1 ppm Ethanol 18 ppm <1 ppm Aceton 58 ppm 2 ppm | 14 °C | 14 bar | 2,6 cm/min | <10 | 78 | max. 78 |
| 7 | B | Grace 10A | 12 t | Isobuten >99% 1-Buten 0,10% 2-Butene 0,20% | Acetonitril 8 ppm <1 ppm Acetaldehyd 3 ppm <1 ppm Methanol 9 ppm <1 ppm Ethanol 19 ppm <1 ppm Aceton 53 ppm <1 ppm | 14 °C | 14 bar | 0,6 cm/min | < 10 | 1330 | max. 1330 |
| 8 | B | Grace 10A | 12 t | Isobuten >99% 1-Buten 0,10% 2-Butene 0,20% | Acetonitril 6 ppm <1 ppm Acetaldehyd 5 ppm <1 ppm Methanol 13 ppm <1 ppm Ethanol 17 ppm <1 ppm Aceton 61 ppm 3 ppm | 35 °C | 14 bar | 2,6 cm/min | < 10 | 2708 | max. 2708 |

**Patentansprüche**

1. Verfahren zur Verringerung des Gehalts an sauerstoffhaltigen und/oder stickstoffhaltigen Verbindungen in Stoffströmen mit einem Isobuten-Gehalt von wenigstens 10 Gew.%, bei dem man den Stoffstrom in flüssiger Phase bei einer Temperatur T [in K] und mit einer linearen Geschwindigkeit v [in cm/min] über ein Festbett eines säurefreien Zeoliths einer mittleren Porengröße von 0,3 bis 1,5 nm leitet, wobei das Festbett in Strömungsrichtung des Stoffstroms eine Ausdehnung I [in cm] aufweist und T, v und I der Gleichung

$$2^{(T-283\ K)/10\ K} \bullet l/v \leq 500\ min$$

gehorchen, wobei T im Bereich von - 25 bis 20 °C liegt

2. Verfahren nach Anspruch 1, wobei v im Bereich von 0,5 bis 35 cm/min liegt.

3. Verfahren nach einem der vorhergehenden Ansprüche, wobei der Stoffstrom außerdem von Isobuten verschiedene Kohlenwasserstoffe enthält.

4. Verfahren nach einem der Ansprüche 1 oder 2, wobei der Stoffstrom außerdem halogenierte Kohlenwasserstoffe enthält.

5. Verfahren nach einem der vorhergehenden Ansprüche, wobei der Zeolith Natriumionen und/oder Calciumionen zur Ladungskompensation enthält.

6. Verfahren nach einem der vorhergehenden Ansprüche, wobei der Zeolith unter Zeolith A, Zeolith L, Zeolith X und Zeolith Y ausgewählt ist.

7. Verfahren nach einem der vorhergehenden Ansprüche, wobei man den Stoffstrom vor der Zeolithbehandlung trocknet.

8. Verfahren nach einem der vorhergehenden Ansprüche, wobei man den Stoffstrom über ein Festbett leitet, das stromaufwärts zur Strömungsrichtung des Stoffstromes einen Zeolith mit einer mittleren Porengröße von 0,3 bis 0,4 nm und stromabwärts einen Zeolith mit einer mittleren Porengröße von wenigstens 0,5 nm umfasst.

9. Verfahren nach einem der vorhergehenden Ansprüche, wobei man den Stoffstrom zur Herstellung von Isobutenpolymeren verwendet.

**Claims**

1. A process for reducing the content of oxygen-containing and/or nitrogen-containing compounds in streams having an isobutene content of at least 10% by weight, which comprises passing the stream in the liquid state at a temperature T [in K] and a linear velocity v [in cm/min] over a fixed bed of an acid-free zeolite having a mean pore size of from 0.3 to 1.5 nm, where the fixed bed has a length 1 [in cm] in the flow direction of the stream and T, v and 1 obey the relationship

$$2^{(T-283\ K)/10\ K} \bullet 1/v \leq 500\ min,$$

wherein T is in the range from -25 to 20°C.

2. The process according to claim 1, wherein v is in the range from 0.5 to 35 cm/min.

3. The process according to any of the preceding claims, wherein the stream further comprises hydrocarbons other than isobutene.

**4.** The process according to claim 1 or 2, wherein the stream further comprises halogenated hydrocarbons.

**5.** The process according to any of the preceding claims, wherein the zeolite comprises sodium ions and/or calcium ions to balance the charge.

**6.** The process according to any of the preceding claims, wherein the zeolite is selected from among zeolite A, zeolite L, zeolite X and zeolite Y.

**7.** The process according to any of the preceding claims, wherein the stream is dried prior to the zeolite treatment.

**8.** The process according to any of the preceding claims, wherein the stream is passed through a fixed bed which comprises a zeolite having a mean pore size of from 0.3 to 0.4 nm upstream relative to the flow direction of the stream and a zeolite having a mean pore size of at least 0.5 nm downstream.

**9.** The process according to any of the preceding claims, wherein the stream is used for preparing isobutene polymers.

## Revendications

**1.** Procédé de diminution de la teneur en composés contenant de l'oxygène et/ou contenant de l'azote dans des courants de substance ayant une teneur en isobutène d'au moins 10 % en poids, dans lequel le courant de substance est dirigé en phase liquide sur un lit fixe d'une zéolithe exempte d'acide d'une taille de pore moyenne de 0,3 à 1,5 nm, à une température T [en K] et avec une vitesse linéaire v [en cm/min], le lit fixe présentant une extension I [en cm] dans la direction d'écoulement du courant de substance et T, v et I se conformant à l'équation

$$2^{(T-283\ K)/10\ K} \bullet I/v \leq 500\ min$$

dans laquelle T se situe dans la plage de -25 à 20 °C.

**2.** Procédé selon la revendication 1, dans lequel v se situe dans la plage de 0,5 à 35 cm/min.

**3.** Procédé selon l'une des revendications précédentes, dans lequel le courant de substance contient en outre des hydrocarbures différents de l'isobutène.

**4.** Procédé selon l'une des revendications 1 ou 2, dans lequel le courant de substance contient en outre des hydrocarbures halogénés.

**5.** Procédé selon l'une quelconque des revendications précédentes, dans lequel la zéolithe contient des ions sodium et/ou des ions calcium pour la compensation de charge.

**6.** Procédé selon l'une quelconque des revendications précédentes, dans lequel la zéolithe est sélectionnée parmi la zéolithe A, la zéolithe L, la zéolithe X et la zéolithe Y.

**7.** Procédé selon l'une quelconque des revendications précédentes, dans lequel le courant de substance sèche avant le traitement par zéolithe.

**8.** Procédé selon l'une quelconque des revendications précédentes, dans lequel le courant de substance est dirigé sur un lit fixe qui comporte, en amont par rapport à la direction d'écoulement du courant de substance, une zéolithe ayant une taille de pore moyenne de 0,3 à 0,4 nm, et en aval, une zéolithe ayant une taille de pore moyenne d'au moins 0,5 nm.

**9.** Procédé selon l'une quelconque des revendications précédentes, dans lequel le courant de substance est utilisé pour la préparation de polymères d'isobutène.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9310063 A
- EP 0628575 B1
- DE 3914817 A
- SU 1011624 A
- EP 582901 A

**In der Beschreibung aufgeführte Nicht-Patentliteratur**

- Ullmanns Enzyklopädie d. Techn. Chemie. 1983, vol. 17, 9-17